(19) Europäisches Patentamt
European Patent Office
Office européen des brevets

(11) Veröffentlichungsnummer: **0 339 454 A2**

(12) **EUROPÄISCHE PATENTANMELDUNG**

(21) Anmeldenummer: **89107011.2**

(22) Anmeldetag: **19.04.89**

(51) Int. Cl.4: **C07D 309/40 , C07C 97/03 , C07C 69/78**

(30) Priorität: **27.04.88 CH 1569/88**

(43) Veröffentlichungstag der Anmeldung: **02.11.89 Patentblatt 89/44**

(84) Benannte Vertragsstaaten: **AT BE CH DE FR GB IT LI NL**

(71) Anmelder: **L. GIVAUDAN & CIE Société Anonyme**
**CH-1214 Vernier-Genève(CH)**

(72) Erfinder: **Wild, Hans-Jakob, Dr.**
**Sonnenbergstrasse 13a**
**CH-8633 Wolfhausen(CH)**

(74) Vertreter: **Urech, Peter, Dr. et al**
**Grenzacherstrasse 124 Postfach 3255**
**CH-4002 Basel(CH)**

(54) **Verfahren zur Herstellung von ungesättigten, cyclischen Ketonen.**

(57) Die Erfindung betrifft ein Verfahren zur Herstellung von γ-Pyronen, und zwar der Verbindungen der Formel

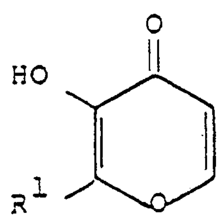

I

worin $R^1$ Methyl oder Aethyl bedeutet.
Diese γ-Pyrone der Formel I sind bekannte Riech- und Geschmackstoffe.
Das Verfahren ist dadurch gekennzeichnet, dass man eine Verbindung der Formel

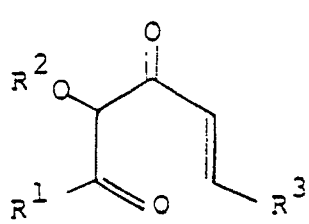

II

worin $R^1$ obige Bedeutung besitzt, $R^2$ Alkanoyl oder, gegebenenfalls substituiertes Benzoyl, und $R^3$ für den Rest

EP 0 339 454 A2

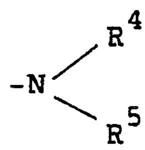

worin $R^4$ und $R^5$ nieder Alkyl, insbesondere Methyl, darstellt, oder für die Hydroxygruppe steht, einem Pyronringschluss in saurem Milieu unterzieht, und, dass man das Reaktionsprodukt der Formel

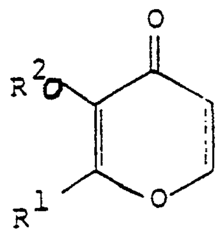

III

worin $R^1$ und $R^2$ obige Bedeutung besitzen,
alkalisch hydrolysiert.

# Verfahren zur Herstellung von ungesättigten, cyclischen Ketonen

Die Erfindung betrifft ein Verfahren zur Herstellung von γ-Pyronen, und zwar der Verbindungen der Formel

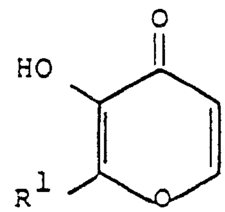

I

worin $R^1$ Methyl oder Aethyl bedeutet.

Diese γ-Pyrone der Formel I sind bekannte Riech- und Geschmackstoffe.

Das Verfahren ist dadurch gekennzeichnet, dass man eine Verbindung der Formel

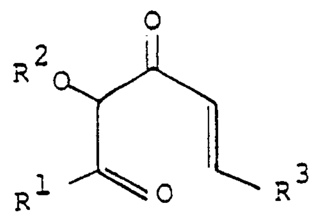

II

worin $R^1$ obige Bedeutung besitzt, $R^2$ Alkanoyl oder, gegebenenfalls substituiertes Benzoyl, und $R^3$ für den Rest

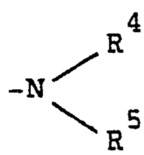

worin $R^4$ und $R^5$ nieder Alkyl, insbesondere Methyl, darstellt, oder für die Hydroxygruppe steht, einem Pyronringschluss in saurem Milieu unterzieht, und, dass man das Reaktionsprodukt der Formel

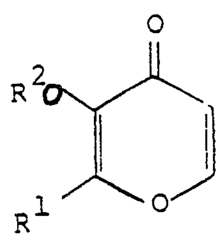

III

worin $R^1$ und $R^2$ obige Bedeutung besitzen, alkalisch hydrolysiert.

Die geeigneten Alkanoylreste sind die Säurereste von nieder-Alkancarbonsäuren, insbesondere von solchen mit 1-6 Kohlenstoffatomen; Beispiele sind Essigsäure, Propionsäure, Buttersäure, Isobuttersäure, etc.

Die geeigneten Substituenten von Benzoyl sind nieder-Alkyl nieder-Alkoxy, Halogen, Nitro, Alkanoyloxy. Bevorzugt sind unsubstituiertes Benzoyl und P-Toluyl.

Die nieder-Alkylreste von $R^4$, $R^5$ und $R^6$ - die gleich oder verschieden sein können enthalten zweckmässigerweise 1-6 Kohlenstoffatomene. Sie können - geradkettig oder verzweigt sein. Beispiele sind

Methyl, Aethyl, Propyl, i-Propyl, Butyl, Hexyl.

Der Pyronringschluss der Verbindung II wird zweckmässigerweise in leicht oder mässig saurem Milieu durchgeführt, geeignete Säuren sind Alkancarbonsäuren, z.B. Ameisensäure, Essigsäure, Propionsäure, Oxalsäure, verdünnte Mineralsäuren wie verdünnte Salzsäure oder verdünnte Schwefelsäure.

Als Lösungsmittel dient zweckmässigerweise die Säure.

Die Temperatur ist nicht kritisch, sie kann z.B. zwischen 0 und 100 °C liegen.

Die Hydrolyse von III wird alkalisch, zweckmässigerweise in alkalischem Milieu oberhalb pH ca. 10 durchgeführt. Die Reaktionstemperatur ist zweckmässigerweise die Raumtemperatur.

Die Verbindung II wird erfindungsgemäss aus einer Verbindung

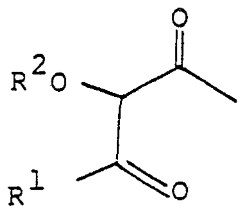

IV

durch deren Kettenverlängerung um ein Kohlenstoffatom mittels eines Ameisensäurederivats

H C -OR$^6$ V
‖
X

worin X Sauerstoff ist oder

C für 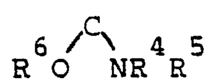
‖
X

steht, wobei R$^6$ nieder-Alkyl ist,
erhalten.

Die experimentelle Durchführung dieser Claisenkondensation hängt von der Natur des Substituenten X ab, und zwar gemäss folgender Tabelle:

## Herstellung der Verbindung II

| Verbindung V | Reaktionstyp | Reagens | Temp. | Literatur |
|---|---|---|---|---|
| HCOR$^6$ ‖ O | Claisenkondensation | starke Basen z.B. Alkaliamide, wie Lithiumdiisopropylamid | -78°C bis 50°C | H.O. House, Modern Synthetic Reactions, W.A. Benjamin, Menlo Pk, Calif., 2nd ed., 1972, 629 seq. |
| H COR$^6$ /\ RO NR$^4$R$^5$ | Aminomethylenierung von IV | $R^4R^5$N-CH(OR$^6$) | 20°C bis 100°C | R.F. Abdulla, Tetrahedron 35, (1979) 1675 |

Anstelle von IV kann selbstverständlich auch dessen Enolat eingesetzt werden.

Der leicht zu bewerkstelligende Ringschluss II → III ist überraschend.

Zwar ist durch M. Koreeda, Tet. Letters (1980), 1197 der Ringschluss zu einem γ-Pyron gemäss:

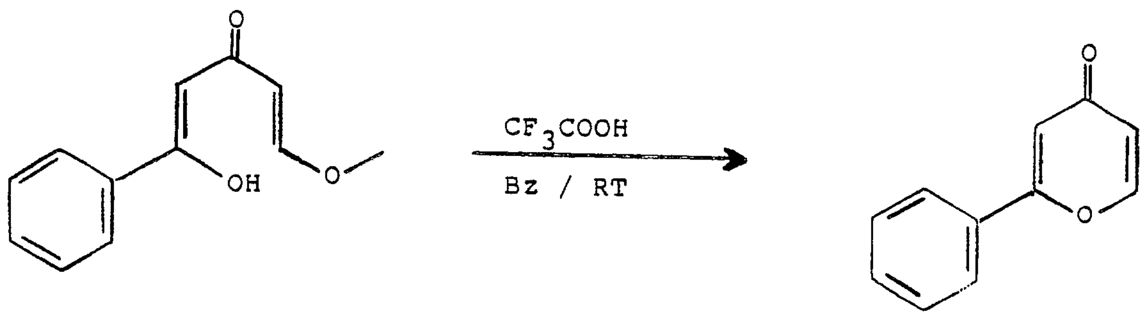


bekanntgeworden.

Im Falle der Verbindung II war wegen des Vorhandenseins des Substituenten $R^2O$ viel eher folgender Ablauf, führend zu Isomaltol vorauszusehen:

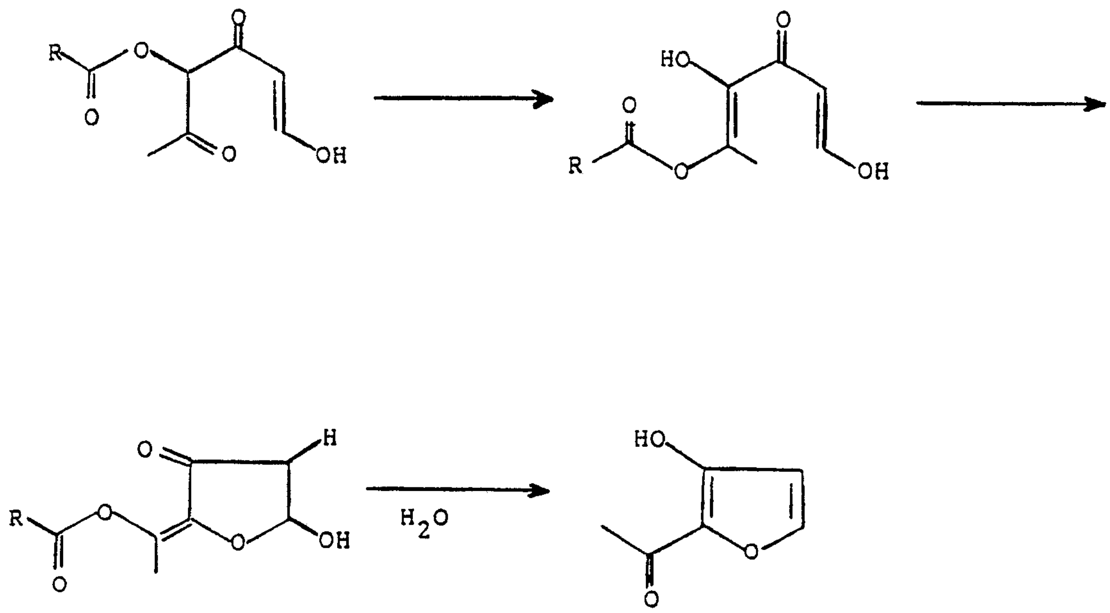


oder - sogar eine Hydrolyse des Esters hätte ernsthaft in Betracht gezogen werden müssen:

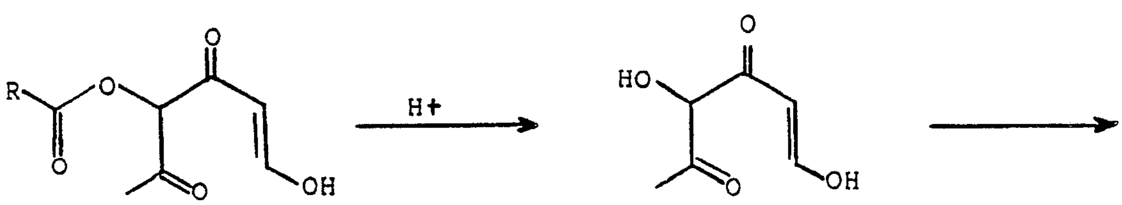


Isomaltol.

## Beispiel 1

a) 30,04 g (300 mMol) Acetylaceton werden unter Stickstoff vorgelegt und bei 0 °C innert 3 Stunden mit 40,50 g (100 mMol) Sulfurylchlorid versetzt. Nach Ende der Gasentwicklung wird auf Raumtemperatur erwärmt und 30 Minuten nachgerührt. Das Reaktionsgemisch wird direkt bei 40-44 °C/20 mbar destilliert. Es resultieren 29,98 g (74%) 3-Chlor-4-hydroxy-3-penten-2-on.

| | |
|---|---|
| IR (fl. Film): | 1725m, 1605s, 1400m, 1040m, 910m $cm^{-1}$ |
| NMR 60 MHz ($CHCl_3$): | 2,3 (s,6H), 15,6 (s,1H) ppm |
| MS (m/e): | 134 ($M^+$), 119, 99, 92, 43 (100%) |

b) 79,20 g (550 mMol) Natriumbenzoat werden in 1 l N,N-Dimethylformamid suspendiert. Man lässt hierauf innert 10 Minuten 67,28 g (500 mMol) 3-Chlor-4-hydroxy-3-penten-2-on zulaufen und rührt 3 Stunden bei 20-25°C nach. Das Reaktionsgemisch wird auf 500 ml 1N HCl gegossen und im Scheidetrichter mit dreimal je 500 ml Aethylacetat extrahiert. Die organischen Phasen werden mit 500 ml gesättigter Natriumbicarbonatlösung gewaschen, dann vereinigt, über Magnesiumsulfat getrocknet und am Rotationsverdampfer eingeengt. Das Rohprodukt destilliert bei 106-110°C/0,04 mbar. Es resultieren 77,94 g (71%) eines Tautomerengemisches von 3-Benzoyloxy-2,4-pentandion + 3-Benzoyloxy-4-hydroxy-3-penten-2-on im Verhältnis 3:1.

| | |
|---|---|
| IR (fl. Film): | 3420w, 1740s (Schulter), 1720s, 1270s, 1110s, 710 $cm^{-1}$ |
| NMR (400 mHz ($CDCl_3$): | 2,1 (s/6H), 2,4 (s/6H), 5,75 (s/1H), 7,5-8,4 (m/5H), 14,6 (s/1H) ppm |
| MS (m/e): | 220 ($M^+$), 178, 105 (100%), 77, 43 |

c1) 44,00 g (200 mMol) 3-Benzoyloxy-2,4-pentandion werden auf 40°C erwärmt. Innert 3 Stunden lässt man 58,89 g (400 mMol) N,N-Dimethylformamiddiäthylacetal zulaufen. Anschliessend wird noch 3 Stunden bei 40°C nachgerührt. Das Reaktionsgemisch wird am Rotationsverdampfer eingeengt, dann in 400 ml Aethylacetat gelöst und im Scheidetrichter mit 200 ml 10%iger $NaH_2PO_4$-Lösung und dann mit 200 ml gesättigter NaCl-Lösung gewaschen. Die organische Phase wird über Magnesium sulfat getrocknet und am Rotavapor eingeengt. Das Rohprodukt wird in Aethylacetat/Hexan 4:1 über Kieselgel drucksäulenchromatographiert. Es resultieren 26,37 g (48%) 4-Benzoyloxy-1-dimethylamino-1-hexen-3,5-dion.

| | |
|---|---|
| IR (fl. Film): | 1730m, 1715m, 1645m, 1575s $cm^{-1}$ |
| NMR 60 MHz ($CDCl_3$): | 2,38 (s/3H), 3,02 (d/6H), 5,38 (d/1H), 5,75 (s/1H), 7,78 (d/1H), 7,30-8,24 (m/5H) ppm |
| MS (m/e): | 275 ($M^+$), 170, 105, 98 (100%), 77, 42 |

dl) 24,75 g (90 mMol) 4-Benzoyloxy-1-dimethylamino-1-hexen-3,5-dion werden in 180 ml Essigsäure während 90 Minuten rückflussiert. Das Reaktionsgemisch wird am Rotationsverdampfer bei 30-50°C eingeengt, der Rückstand in 200 ml Dichlormethan gelöst und mit zweimal je 100 ml gesättigter Natriumbicarbonatlösung gewaschen. Die organische Phase wird über Magnesiumsulfat getrocknet, am Rotationsverdampfer eingeengt und ergibt 20,11 g (97.1%) 3-Benzoyloxy-2-methyl-4-pyron. Gehalt 86%, SmP. 106-110°C.

| | |
|---|---|
| IR (fl. Film). | 3000m, 1745s, 1660s $cm^{-1}$ |
| NMR 60 MHz ($CDCl_3$): | 2,30 (s/3H), 6,48 (d/1H), 7,78 (d/1H), 7,30-8,40 (m/5H) ppm |
| MS (m/e): | 230 ($M^+$), 105 (100%), 77, 43 |

e1) 19,00 g (82,6 mMol) 3-Benzoyloxy-2-methyl-4-pyron werden mit 124 ml (248 mMol) 2N Natronlauge während 3 Stunden bei 20-25°C verrührt. Die Lösung wird auf pH 6,5 gestellt und dreimal mit je 100 ml Dichlormethan extrahiert. Die vereinigten organischen Phasen werden über Magnesiumsulfat getrocknet und am Rotationsverdampfer eingeengt. Das Rohprodukt wird in Aceton umkristallisiert und ergibt 7,21 g (69.3%) 3-Hydroxy-2-methyl-4-pyron, Smp. 157-158°C.

| | |
|---|---|
| IR ($CHCl_3$): | 3420w, 3000w, 1675m, 1630s, 1570m $cm^{-1}$ |
| NMR 60 MHz ($CDCl_3$): | 2,37 (s/3H), 6,45 (d/1H), 6,85 (s, breit/1H), 7,67 (d/1H) ppm |
| MS (m/e): | 126 ($M^+$, 100%), 97, 71, 55, 43 |

Beispiel 2

c2) 48,6 g (480 mMol) Diisopropylamin werden in 350 ml Tetrahydrofuran gelöst. Bei -40° C tropft man 275 ml (440 mMol) 1,6 molare Butyllithium-Lösung in Hexan zu. Dann kühlt man auf -78° C ab und tropft innert 30 Minuten 44,02 g (200 mMol) 3-Benzoyloxy-2,4-pentandion in 50 ml Tetrahydrofuran zu. Anschliessend tropft man bei -78° C innert 30 Minuten 22,20 g (300 mMol) Aethylformiat zu und rührt 2 Stunden nach. Das Reaktionsgemisch wird auf 600 ml 1N Salzsäure gegossen, mit Natriumchlorid gesättigt und mit dreimal je 500 ml Aethylacetat extrahiert. Die vereinigten organischen Phasen werden über Magnesiumsulfat getrocknet, am Rotationsverdampfer eingeengt und ergeben 54,30 g 4-Benzoyloxy-1-hydroxy-1-hexen-3,5-dion.

| | |
|---|---|
| IR ($CHCl_3$): | 3000m, 1725s, 1625m $cm^{-1}$ |
| MS (m/e): | 205, 178, 148, 122, 105 (100%), 77, 51, 43 |

d2) 54,20 g (218 mMol) 4-Benzoyloxy-1-hydroxy-1-hexen-3,5-dion werden in 400 ml Essigsäure während 2 Stunden rückflussiert. Das Reaktionsgemisch wird am Rotationsverdampfer bei 40° C eingeengt, der Rückstand in 500 ml Dichlormethan gelöst und zweimal mit 300 ml gesättigter Natriumbicarbonatlösung gewaschen. Die organische Phase wird über Magnesiumsulfat getrocknet, am Rotationsverdampfer eingeengt und ergibt 40.75 g (81,2%) 3-Benzoyloxy-2-methyl-4-pyron, Gehalt 73%, Smp.; 106-110° C.

| | |
|---|---|
| IR ($CHCl_3$): | 3000m, 1740s, 1660s $cm^{-1}$ |
| NMR 60 mHz ($CDCl_3$): | 2,30 (s/3H), 6,48 (d/1H), 7,78 (d/1H), 7,30-8,40 (m/5H) ppm |
| MS (m/e): | 230 ($M^+$), 105 (100%), 77, 43 |

e2) 40,60 g (176,5 mMol) 3-Benzoyloxy-2-methyl-4-pyron werden mit 265 ml (530 mMol) 2N Natronlauge während 3 Stunden bei 20-25° C verrührt. Die Lösung wird auf PH 6,5 gestellt und dreimal mit je 200 ml Dichlormethan extrahiert. Die vereinigten organischen Phasen werden über Magnesiumsulfat getrocknet und am Rotationsverdampfer eingeengt. Das Rohprodukt wird in Aceton umkristallisiert und ergibt 8,90 g (40%) 3-Hydroxy-2-methyl-4-pyron, Smp. 156-158° C.

| | |
|---|---|
| IR ($CHCl_3$): | 3420w, 3000w, 1670m, 1630s, 1570m $cm^{-1}$ |
| NMR 60 mHz ($CDCl_3$): | 2,37 (s/3H), 6,45 (d/1H), 6,75 (s, breit/1H), 7,67 (d/1H) ppm |
| MS (m/e): | 126 ($M^+$, 100%) 97, 71, 55, 43 |

## Ansprüche

1. Verfahren zur Herstellung von Verbindungen der Formel

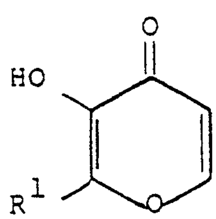

I

worin $R^1$ Methyl oder Aethyl bedeutet,
dadurch gekennzeichnet, dass man eine Verbindung der Formel

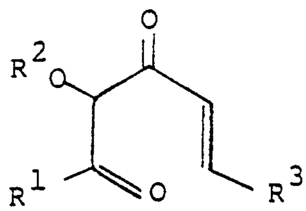

II

worin $R^1$ obige Bedeutung besitzt, $R^2$ Alkanoyl oder gegebenenfalls substituiertes Benzoyl und $R^3$ für den Rest

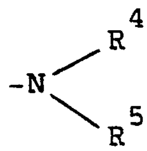

worin $R^4$ und $R^5$ nieder-Alkyl darstellt, oder für die Hydroxygruppe steht,
einem Pyronringschluss in saurem Milieu unterzieht, und, dass man das Reaktionsprodukt der Formel

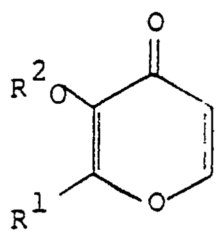

III

worin $R^1$ und $R^2$ obige Bedeutung besitzen,
alkalisch hydrolysiert.

2. Verfahren nach Anspruch 1, dadurch gekennzeichnet, dass man die Verbindung der Formel II durch Umsetzung einer Verbindung der Formel

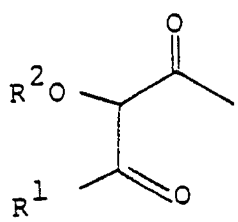

IV

mit einer Verbindung der Formel

H C -OR$^6$ V
‖
X

worin X Sauerstoff ist oder

C den Rest 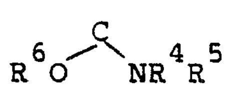
‖
X

darstellt, wobei $R^6$ nieder-Alkyl ist,
gewinnt.

3. Verfahren nach Anspruch 1 oder 2, dadurch gekennzeichnet, dass $R^2$ Benzoyl ist.

4. Verfahren nach Anspruch 1, 2 oder 3, dadurch gekennzeichnet, dass $R^1$ Methyl ist.

5. Verfahren nach Anspruch 1, 2 oder 3, dadurch gekennzeichnet, dass $R^1$ Aethyl ist.

6. Verbindungen der Formel

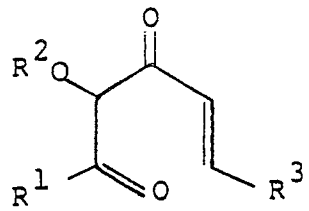

II

worin $R^1$ Methyl oder Aethyl darstellt, $R^2$ Alkanoyl oder gegebenenfalls substituiertes Benzoyl bedeutet, und $R^3$ für den Rest

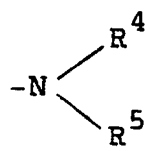

steht, worin $R^4$ und $R^5$ nieder-Alkyl bedeuten, oder für die Hydroxygruppe steht.

7. 4-Benzoyloxy-1-dimethylamino-1-hexen-3,5-dion.

8. 4-Benzoyloxy-1-hydroxy-1-hexen-3,5-dion.